(19) Europäisches Patentamt / European Patent Office / Office européen des brevets

(11) **EP 4 516 248 A1**

(12) **EUROPEAN PATENT APPLICATION**
published in accordance with Art. 153(4) EPC

(43) Date of publication:
**05.03.2025 Bulletin 2025/10**

(21) Application number: **23795350.0**

(22) Date of filing: **24.04.2023**

(51) International Patent Classification (IPC):
**A61B 17/3203** (2006.01)    **A61B 34/10** (2016.01)
**A61B 34/20** (2016.01)

(86) International application number:
**PCT/CN2023/090360**

(87) International publication number:
**WO 2023/207917 (02.11.2023 Gazette 2023/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.04.2022 CN 202210433774**

(71) Applicant: **Healinno (Beijing) Medical Technology Co., Ltd.**
**Beijing 100176 (CN)**

(72) Inventors:
• CHEN, Wenbo
  Beijing 100176 (CN)
• ZHAO, Jing
  Beijing 100176 (CN)
• SHI, Yilun
  Beijing 100176 (CN)
• SHI, Ce
  Beijing 100176 (CN)

(74) Representative: **Zaboliene, Reda**
**Metida**
**Business center Vertas**
**Gyneju str. 16**
**01109 Vilnius (LT)**

(54) **AUTOMATIC WATER JET CUTTER SYSTEM**

(57) The present invention provides an automatic water jet cutter system, wherein: a pre-planned continuous boundary position track is fitted on a navigation image to generate a motion control position track; a water jet cutter coordinate system is established, the motion control position track is converted into the water jet cutter coordinate system, and then motion position track parameters of each axis are calculated; according to the motion position track parameters of each axis, a multi-axis linkage control method is used to control a water jet cutter to perform corresponding motions on a jet flow axis, a suction flow axis, a linear motion axis and a rotary motion axis. In the multi-axis linkage control method, an error of the water jet cutter on any one axis among the jet flow axis, the suction flow axis, the linear motion axis and the rotary motion axis is related to errors on the other three axes. The present application further provides a device used for implementing the method. The problems that existing water jet cutters have rough motion boundaries and motions on various dimensions which cannot be controlled in a linked manner are solved.

Fit a continuous boundary position trajectory pre-planned on the ultrasound image to generate a motion control position trajectory — 101

Establish a water jet cutter coordinate system in real time according to position parameters of the water jet cutter, convert the motion control position trajectory into the water jet cutter coordinate system and then calculate motion position trajectory parameters for each axis — 102

According to the motion position trajectory parameters for each axis, control the jet flow, the suction flow, the linear motion trajectory and the rotary motion trajectory of the water jet cutter by a multi-axis linkage PID control method — 103

FIG. 2(a)

EP 4 516 248 A1

**Description**

**CROSS-REFERENCE TO RELATED APPLICATIONS**

The present application claims priority to Chinese patent application No. 202210433774.8, filed on April 24, 2022, the entire contents of which are incorporated herein by reference.

**TECHNICAL FIELD**

The present invention relates to the technical field of medical electronics, and in particular to an automatic water jet cutter implementing method and device.

**BACKGROUND**

An existing automatic water jet cutter planning method is rough in adjusting motions, and a specific implementable method is not provided to establish an accurate corresponding relationship between a sagittal image and a cross-sectional image obtained by a biplanar ultrasonic probe or other methods. Actually, an operator needs to manually obtain limited ultrasound image information and perform piecewise (e.g., in three to four segments) position matching and parameter setting of the sagittal plane and the cross-sectional plane. The middle of each segment only can be fitted through interpolation simulation, thus requiring less precision in water jet cutter motion control. In addition, in an existing method for controlling an automatic water jet cutter system, there is no unified and coordinated control of influence and dependence of the high-pressure jet flow, the suction flow, and mechanical motions in all directions to each other. Non-synchronization of various motions will result in the position and depth of action, angle and pressure of the jet in the trajectory planning not being well matched in actual execution due to motion errors, and lead to the problem of large errors in the water jet cutter motion trajectory.

**SUMMARY**

The present invention provides an automatic water jet cutter implementing method and device, thereby solving the problems of the rough motion boundary of existing water jet cutter and the inability to perform linkage control of motions in various dimensions leading to errors in the water jet cutter motion trajectory.

To solve the above problems, the present invention is implemented as follows.

According to a first aspect, the present invention provides an automatic water jet cutter implementing system, including

an imaging module, configured to generate a navigation image;

an image planning module, configured to fit a continuous boundary position trajectory pre-planned on the navigation image to generate a motion control position trajectory, and convert the motion control position trajectory into a water jet cutter coordinate system, then perform calculation and send motion position trajectory parameters for each axis corresponding to water jet cutter jet action points;

a motion control module, configured to receive the motion position trajectory parameters for each axis, generate and send motion position trajectory parameters in a linear motion axis and a rotary motion axis to a water jet cutter head by a multi-axis linkage control method, and send motion position control parameters in a jet flow axis and a suction flow axis to a pipeline and hydraulic power module, and further configured to acquire the motion position trajectory parameters for each axis of the water jet cutter head for closed-loop control of the motion trajectory;

a pipeline and hydraulic power module, configured to transfer liquid to the water jet cutter head according to the motion position control parameters in the jet flow axis sent by the motion control module and perform a suction motion according to the motion position control parameters in the suction flow axis; and

a water jet cutter head, configured to perform respective motions according to the motion position control parameters in the linear motion axis and the rotary motion axis sent by the motion control module, and the motion position control parameters in the jet flow axis,

where in the multi-axis linkage control method, an error in any one of the jet flow axis, the suction flow axis, the linear motion axis and the rotary motion axis of the water jet cutter are correlated with errors in the other three axes.

Further, the motion control position trajectory includes a jet effective length, a jet long-axis position and a cross-sectional angle; and the motion position trajectory parameters for each axis include an action point jet length in the water jet cutter coordinate system, an action point cross-sectional angle, and further include an action point jet long-axis position and/or an action point jet long-axis velocity.

Further, in the multi-axis linkage control method, closed-loop control of a trajectory planning position loop is added, and an error value of the trajectory planning position loop is a difference between a boundary position on the continuous boundary position trajectory at the current moment of measurement and an action point trajectory position obtained by actual measurement.

Further, in the multi-axis linkage control method, errors in the jet flow axis, the suction flow axis, the linear motion axis and the rotary motion axis of the water jet cutter are expressed as Equation 11 to Equation 14.

Preferably, the method further includes: taking the key point image positions on a pre-designed navigation image as accurate information when fitting the continuous boundary position trajectory.

Preferably, the method further includes: performing piecewise fitting of the continuous boundary position trajectory to generate motion control position trajectories for each segment combined to form the motion control position trajectory.

Preferably, the continuous boundary position trajectory is divided into several motion voxels, and each of the motion voxels is interpolated in accordance with a motion trajectory when the water jet cutter is actually operated so as to generate the motion control position trajectory; and each of the motion voxels includes a jet effective length, a jet long-axis position and a cross-sectional angle.

Preferably, the motion control position trajectory is converted into a spatial position trajectory in the water jet cutter coordinate system through a coordinate transform and by an incremental control method, and then motion position trajectory parameters for each axis are calculated.

Preferably, the navigation image is a biplanar ultrasound image or a three-dimensional ultrasound image or a three-dimensional image.

Further, motion position parameters in the jet flow axis, the linear motion axis and the rotary motion axis of the water jet cutter and positions of the continuous boundary position trajectory in the water jet cutter coordinate system are acquired in real time, and corresponding coordinates are subtracted to obtain an error value of the trajectory planning position loop.

Further, the motion position trajectory parameters in the jet flow axis of the water jet cutter acquired in lag time are combined with the motion position trajectory parameters in the rotary motion axis and the linear motion axis of the water jet cutter acquired in real time, to perform closed-loop control of a trajectory planning position loop by a time-lag system regulation method.

Further, piecewise fitting of the continuous boundary position trajectory is performed by using stepped line segments to obtain the motion control position trajectories for each segment.

Further, piecewise fitting of the continuous boundary position trajectory is performed by a straight line segment interpolation fitting method to obtain the motion control position trajectories for each segment.

Preferably, when motion position trajectory parameters in the jet flow axis of the water jet cutter are acquired in lag time, a cross-sectional image of the same position acquired in advance and a cross-sectional navigation image acquired at the current moment are subtracted and filtered to obtain a high-frequency image signal; and after performing enhancement and boundary excision of the high-frequency graphic signal, measurement is performed to obtain the motion position trajectory parameters in the jet flow axis of the water jet cutter.

Preferably, the time-lag system regulation method includes a Smith prediction control method and/or a fuzzy control method.

At least one technical solution used by the embodiments of the present application can achieve the following beneficial effects:

based on the need for high-precision trajectory planning, the present invention adopts a number of innovative designs for unified motion control, that can optimize a process for planning an automatic water jet cutter, improve the precision of water jet cutter control, help to increase an effective action volume, reduce water jet cutter motion error, and enhance effect and safety of the water jet cutter use.

## BRIEF DESCRIPTION OF THE DRAWINGS

The accompanying drawings described herein are used to provide a further understanding of the present invention, and constitute a part of the present invention. The exemplary embodiments of the present invention and descriptions thereof are used to explain the present invention, and do not constitute inappropriate limitations to the present invention. In the drawings:

FIG. 1(a) is a schematic structural diagram of an apparatus of a system embodiment of the present application;

FIG. 1(b) is a schematic diagram of a piecewise fitting method of a system embodiment of the present application;

FIG. 1(c) is a schematic diagram of another piecewise fitting method of a system embodiment of the present application;

FIG. 1(d) is a schematic diagram of key point images of a system embodiment of the present application;

FIG. 1(e) is a schematic diagram of a water jet cutter coordinate system of a system embodiment of the present application;

FIG. 1(f) is a schematic diagram of a biplanar ultrasound image in a water jet cutter coordinate system of a system embodiment of the present application;

FIG. 1(g) is a schematic diagram of combined biplanar ultrasound images of a system embodiment of the present application;

FIG. 1(h) is a schematic diagram of end faces of a cavity of a water jet cutter of a system embodiment of the present application;

FIG. 2(a) is a method flowchart of a method embodiment of the present application;

FIG. 2(b) is a schematic diagram of a Z-shaped excision trajectory of a method embodiment of the present application;

FIG. 2(c) is a schematic diagram of PID linkage control of a method embodiment of the present application; and

FIG. 2(d) is a schematic diagram of PID time-lag control of a method embodiment of the present application.

## DETAILED DESCRIPTION

To make the objectives, technical solutions and advantages of the present invention clearer, the technical solutions of the present invention will be described clearly and completely below with reference to specific embodiments of the present invention and corresponding accompanying drawings. Apparently, the described embodiments are merely some rather than all of the embodiments of the present invention. All other embodiments obtained by a person of ordinary skill in the art based on the embodiments of the present invention without creative efforts shall fall within the protection scope of the present invention.

The present invention has the following innovation points: first, the present invention proposes a method for generating a motion control position trajectory of a navigation image, where a cross-sectional position and a cross-sectional angle in the navigation image and a jet effective action distance are planned into the motion control position trajectory, and a positional relationship between the motion control position trajectory and a water jet cutter motion coordinate system is established, so that a deviation between an actual position and a planned position of the water jet cutter can be accurately obtained in the motion process of the water jet cutter. Second, the present invention proposes a multi-dimensional motion joint control method, so that automatic linkage control of a jet flow, a suction flow, a linear motion trajectory and a rotary motion trajectory of the water jet cutter can be implemented, and the operation error can be reduced.

The technical solution provided by various embodiments of the present invention will be described in detail below with reference to the accompanying drawings.

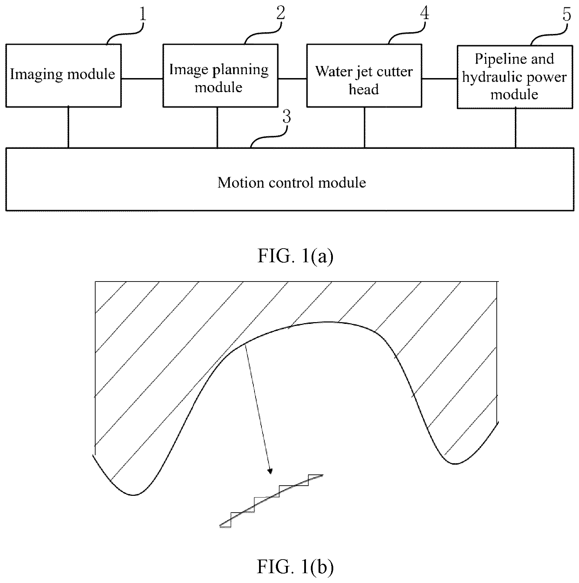

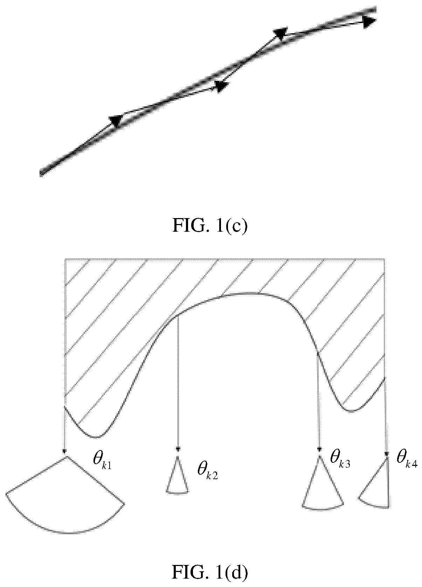

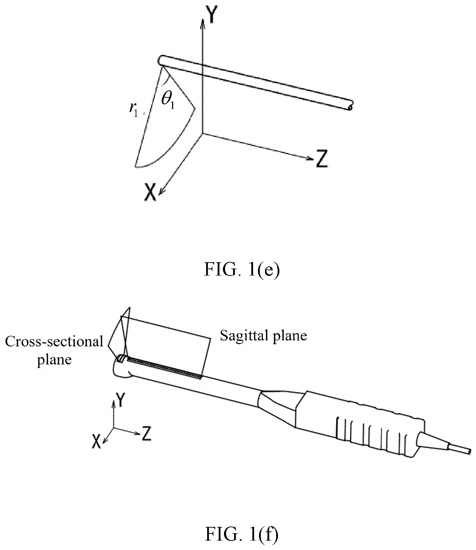

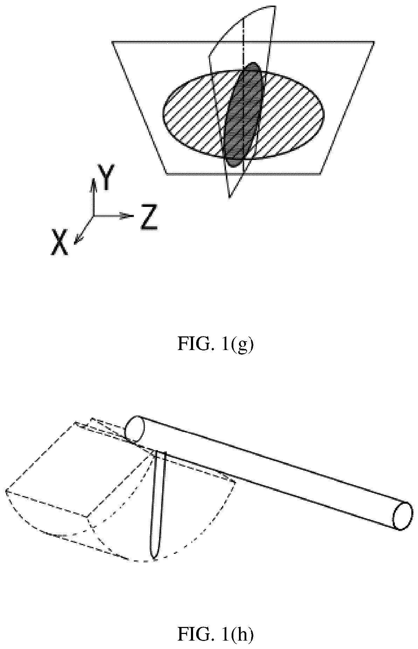

FIG. 1(a) is a schematic structural diagram of an apparatus of a system embodiment of the present application; FIG. 1(b) is a schematic diagram of a piecewise fitting method of a system embodiment of the present application; FIG. 1(c) is a schematic diagram of another piecewise fitting method of a system embodiment of the present application; FIG. 1(d) is a schematic diagram of key point images of a system embodiment of the present application; FIG. 1(e) is a schematic diagram of a water jet cutter coordinate system of a system embodiment of the present application; FIG. 1(f) is a schematic diagram of a biplanar ultrasound image in a water jet cutter coordinate system of a system embodiment of the present application; FIG. 1(g) is a schematic diagram of combined biplanar ultrasound images of a system embodiment of the present application; and FIG. 1(h) is a schematic diagram of end faces of a cavity of a water jet cutter of a system embodiment of the present application.

According to the embodiments of the present invention, a medical automatic water jet cutter includes: an imaging module 1, an image planning module 2, a motion control module 3, a water jet cutter head 4, and a pipeline and hydraulic power module 5.

The imaging module is configured to generate a navigation image.

The image planning module is configured to: fit a continuous boundary position trajectory pre-planned on the navigation image to generate a motion control position trajectory; and establish a water jet cutter coordinate system in real time according to positions of the water jet cutter, convert the motion control position trajectory into the water jet cutter coordinate system and then calculate motion position trajectory parameters for each axis, and send the motion position trajectory parameters for each axis to the motion control module.

The motion position trajectory parameters for each axis include motion position parameters in a linear motion axis, a rotary motion axis, a jet flow axis and a suction flow axis. The motion position parameters in the linear motion axis are an action point jet long-axis position and/or an action point jet long-axis velocity in the water jet cutter coordinate system, the motion position parameters in the rotary motion axis are an action point cross-sectional angle in the water jet cutter coordinate system, and the motion position parameters in the jet flow axis are an action point jet length in the water jet cutter coordinate system.

It should be noted that the action point jet length may be used to indicate that the water jet cutter jet flow and the suction flow are related to the jet flow, for example, the suction flow is equal to the jet flow, or the suction flow at one moment is greater than the jet flow, the suction flow at another moment and so on, that is, the suction flow may be obtained from the jet flow. The respective motion control parameters in the suction flow axis may be obtained from the suction flow.

The motion control module is configured to receive motion position trajectory parameters for each axis of the water jet cutter jet action point, generate and send motion position control parameters in the linear motion axis and the rotary motion axis to motion control elements of the water jet cutter head by a multi-axis linkage PID control method, and send motion position control parameters in the jet flow axis and the suction flow axis to the pipeline and hydraulic power module; and further configured to acquire the motion position trajectory parameters for each axis of the water jet cutter head for closed-loop control of the motion trajectory.

It should be noted that motion position trajectory parameters for each axis represent positions of the water jet cutter head in the water jet cutter coordinate system, and motion position control parameters for each axis are used to control the water jet cutter head or the pipeline and hydraulic power module to perform motions. The motion control module can move the water jet cutter in accordance with motion position trajectory parameters for each axis by a motor or other mechanisms controlling the motion of the water jet cutter head, and the motion control module can move the pipeline and hydraulic power module in accordance with motion position trajectory parameters for each axis by a hydraulic pump or other mechanisms of the pipeline and hydraulic power module.

Preferably, the motion position control parameters in the jet flow axis and the suction flow axis are control parameters converted by the motion position trajectory parameters in the jet flow axis, where the suction flow may be equal to the jet flow.

In the motion position control parameters for each axis, the motion position control parameters and the motion position trajectory parameters in the linear motion axis are the same, and are the action point jet long-axis position and/or the action point jet long-axis velocity in the water jet cutter coordinate system; and the motion position control parameters and the motion position trajectory parameters in the rotary motion axis are the same, and are the action point cross-sectional angle in the water jet cutter coordinate system.

The motion position trajectory parameters in the jet flow axis are action point jet lengths, which are required to be converted into jet flow motion control parameters (motion position control parameters in the jet flow axis), and the conversion relationship is determined by the pipeline mechanical design of the water jet cutter head and the pipeline and hydraulic power module, which may be obtained through experiment.

The motion control parameters in the suction flow axis are obtained by the motion control module according to the control parameters in the jet flow axis, and the suction flow can be kept equal to the jet flow.

The pipeline and hydraulic power module is configured to transfer liquid to the water jet cutter head according to the motion position control parameters in the jet flow axis sent by the motion control module and perform a suction motion according to the motion position control parameters in the suction flow axis.

The water jet cutter head is configured to perform respective motions according to the motion position trajectory parameters in the linear motion axis and the rotary motion axis sent by the motion control module, and the motion position control parameters in the jet flow axis.

In the embodiments of the present invention, the pipeline and hydraulic power module may be configured to suck waste liquid, and the water jet cutter head may be configured to excise an object.

In the embodiments of the present invention, in the multi-axis linkage PID control method, an error in any one of the jet flow axis, the suction flow axis, the linear motion axis and the rotary motion axis of the water jet cutter are correlated with errors in the other three axes; the motion position parameters for each axis are used to indicate respective actions of the water jet cutter head and the pipeline and hydraulic power module in the jet flow axis, the suction flow axis, the linear motion axis and the rotary motion axis.

In the embodiments of the present invention, the motion control module is further configured to control a probe of the imaging module to move, and control the pipeline and hydraulic power module to transfer high-pressure saline to the water jet cutter module and discharge waste liquid in accordance with the suction flow.

In the embodiments of the present invention, a miniaturized laser range finder or hydrophone in synchronized motion with a nozzle may be installed in the water jet cutter head module for obtaining the action point jet long-axis position of the water jet cutter in real time. Other range finders in synchronization with the nozzle may also be installed in the water jet cutter head, which is not particularly limited here.

In the embodiments of the present invention, the imaging module includes: an electric stepper, configured to drive the biplanar ultrasound probe to move to obtain ultrasound images of various positions.

In the embodiments of the present invention, the imaging module further includes a biplanar ultrasound probe and/or a three-dimensional ultrasound probe, where the biplanar probe is configured to generate a biplanar ultrasound image, and the three-dimensional ultrasound probe is configured to generate a three-dimensional ultrasound image. It should be noted that the biplanar probe is driven by the electric stepper to move to obtain a plurality of end-face voxel images with position indexing information, and three-dimensional ultrasound images may also be generated by software for generating three-dimensional images.

The imaging module further includes: a nuclear magnetic resonance image module, configured to obtain a three-dimensional image, for example, a navigational three-dimensional image obtainable from a device such as a nuclear magnetic resonance device.

It should be noted that in specific implementations, dividing each of the above modules according to module functions can be implemented by configuring them in terms of a physical location, a hardware and software/firmware architecture as needed.

In the embodiments of the present invention, the continuous boundary position trajectory may be pre-planned on the three-dimensional ultrasound image or the biplanar ultrasound image, where the continuous boundary position trajectory is an excision trajectory that is planned in advance as needed.

It should be noted that the continuous boundary position trajectory is planned in a base coordinate system which is a human-defined stationary coordinate system, as shown in FIG. 1(f), where a first point when the ultrasound probe is in use may be defined as a coordinate origin, a forward direction of the ultrasound probe is defined as a z-axis positive direction, and a cross-section graph corresponding to the ultrasound probe is an xy plane, and x-axis and y-axis positive directions may be uniquely determined according to the right-handed theorem. It should also be noted that the base coordinate system may also be defined in other ways that are not specifically limited here.

For example, as shown in FIG. 1(b), a projection of a pre-planned continuous position trajectory in the yoz plane is provided. In general, a planned trajectory conforming to the artificial habit is a continuous curve, for example, a shaded area in FIG. 1(b) is a targeted excision area and the lower curve is a manually planned excision trajectory.

Further, the continuous boundary position trajectory includes several motion voxels, where each of the motion voxels includes a jet effective length r, a jet long-axis position z and a cross-sectional angle $\theta$.

In the embodiments of the present invention, the motion control position trajectory is a trajectory generated based on fitting the continuous boundary position trajectory, and is also a trajectory in the base coordinate system. Considering that there will be an error in the operation of the user in the course of actual excision, it is necessary to fit the continuous boundary position trajectory.

In the embodiments of the present invention, the image planning module is further configured to interpolate each of the motion voxels in accordance with a motion trajectory when the water jet cutter is actually operated so as to generate the motion control position trajectory.

Further, trajectory planning of the continuous boundary position trajectory may be performed by a method for fusing biplanar ultrasound image key point information and minimized error interpolation trajectory fitting, that is, with pre-planned key point images as accurate information.

For example, as shown in FIG. 1(d), four key point images when curve-fitting is performed are labeled, where $\theta_{k1}$, $\theta_{k2}$, $\theta_{k3}$, and $\theta_{k4}$ are cross-sectional angles of first to fourth key point images, and a yz-plane position of a corresponding key point image at each cross-sectional angle is fitted with the four key point images as accurate information. It should be noted that the present invention does not specifically limit the number of key point images.

Further, the continuous position trajectory may be curve-fitted by an overall fitting or piecewise fitting method, and the continuous position trajectory may be fitted by a fitting method such as a polynomial or a least-squares approximation, and curve-fitting of the continuous position trajectory may also be implemented by other methods, all of which are not particularly limited here.

It should be noted that if the polynomial fit is used, considering that fitting a complex curve requires a higher polynomial equation, a trajectory is generally generated by using a linear segment approximation of the curve, and a step size is generally determined according to fitting errors when approximating.

Further, when piecewise fitting of the continuous position trajectory is performed, motion control position trajectories for each segment are generated, and the motion control position trajectories are obtained from the motion control trajectories for each segment by piecewise combination.

For example, as shown in FIG. 1(b), piecewise fitting of the pre-planned continuous position trajectory may be performed by using stepped line segments to obtain the motion control position trajectories for each segment. When convenient for control, motors may perform curve-fitting alternately by using one step size for one shaft motor motion while calculating an error for the other shaft motor.

For another example, as shown in FIG. 1(c), piecewise fitting of the continuous position trajectory may also be performed by a straight line segment interpolation fitting method to obtain the motion control position trajectories for each segment. The linear segment interpolation fitting method can be implemented by means of generating linear segments with an arbitrary slope by synchronous motions of two-axis DC brushless motors when controlling, which can effectively reduce fitting errors compared with the stepped fitting method while smoothing and softening the motion trajectory of the motors.

In the embodiments of the present invention, the motion position trajectory parameters for each axis are used to indicate respective actions in the jet flow axis, the suction flow axis, the linear motion axis and the rotary motion axis that are performed by motors in the water jet cutter head and the pipeline and hydraulic power module. The motion position trajectory parameters for each axis include an action point jet length r1 in the water jet cutter coordinate system and an action point cross-sectional angle $\theta_1$, and further include an action point jet long-axis position z1 and/or an action point jet long-axis velocity vz1.

It should be noted that the water jet cutter coordinate system is a motion coordinate system which is transformed in real time according to real time positions of the water jet cutter, and the water jet cutter coordinate system is established with an action point of the water jet cutter head and the excision position as an origin.

As shown in FIG. 1(e), the water jet cutter coordinate system at any moment is an XYZ coordinate system in the figure, and when in motion, the water jet cutter moves along a long axis of the cutter body in a Z-axis positive direction, while the jet reciprocates and rotates in an XY plane perpendicular to the long axis of the cutter body in the range of a given action point cross-sectional angle ($\theta_1$ in the figure), and an action effective distance of the jet on the tissues is shown in the figure as r1. Due to changes in the shape of the action target objects in the course of motion, the Z-direction motion velocity, the size and direction of the action point cross-sectional angle, and the size of the jet action distance are all varied according to the trajectory planning.

FIG. 1(f) is a schematic diagram of two array images of a biplanar ultrasound probe. It can be seen from the figure that in the water jet cutter coordinate system, a direction in which the water jet cutter head moves in a straight line is a +Z direction, and according to a placement position of the ultrasound probe, a plane XY perpendicular to the +Z direction is a cross-section in the water jet cutter coordinate system, and a plane YZ is a sagittal plane in the water jet cutter coordinate system.

FIG. 1(g) is a schematic diagram of combined sagittal and cross-sectional positions in trajectory planning, in the water jet cutter coordinate system XYZ, the sagittal plane and the cross-sectional plane are two planes perpendicular to each other, and a cross-sectional position and a cross-sectional angle may be obtained from an overlapping part of the sagittal plane with the cross-sectional plane.

FIG. 1(h) provides a three-dimensional view of end faces of the cavity after the action of the water jet cutter.

For example, in the process of operating the water jet cutter, a linear motion of the cutter head in the +Z direction, a reciprocating and rotating motion in the XY plane, an action motion of the jet on the object and a suction motion of the waste liquid constitute 4 interrelated simultaneous motion actions. Only unified and coordinated control of the 4 motions can ensure that the water jet accurately excises an object along a planned path to form an irregularly shaped cavity.

At this point, an action point of the water jet excision on the target object may be used as an origin of a tool coordinate system of the executing tool, and through 4-axis linkage control of the above 4 axial motions, required end faces of the cavity may be traced out by the origin of the tool coordinate system by its motion trajectory line in the course of motion, as shown in FIG. 1(h). At this point, two sides of the cavity are determined by angle boundaries of the cross-sectional angle of the jet in the water jet cutter coordinate system in the XY plane, and a width of the jet beam is taken as a progression interval for the tracing action. That is, the jet excision action is converted into a trajectory control problem that the origin of the tool coordinate system traces out end (and side) faces of the cavity through a motion trajectory.

In the embodiments of the present invention, in the multi-axis linkage PID control method adopted by the motion control module, closed-loop control of a trajectory planning position loop is added, and an error value of the trajectory planning position loop is a difference between a boundary position on the continuous boundary position trajectory at the current moment of measurement and an action point trajectory position obtained by actual measurement.

The motion control module may acquire the motion position trajectory parameters in the jet flow axis of the water jet cutter in real time, and may also acquire the motion position trajectory parameters in the jet flow axis of the water jet cutter in lag time.

For example, the motion control module is configured to acquire motion position trajectory parameters in the jet flow axis, the linear motion axis and the rotary motion axis of the water jet cutter in real time, transform the continuous boundary position trajectory into the water jet cutter coordinate system by a coordinate transform, and subtract corresponding coordinates to obtain an error value of the trajectory planning position loop.

For another example, the motion control module is configured to combine the position parameters in the jet flow axis of the water jet cutter acquired in lag time by a cross-sectional image with the position parameters in the rotary motion axis and the linear motion axis of the water jet cutter acquired in real time, and transform the continuous boundary position trajectory into the water jet cutter coordinate system by a coordinate transform to perform closed-loop control of a trajectory planning position loop by a time-lag system PID regulation method.

It should be noted that position parameters in the jet flow axis of the water jet cutter refer to an action point jet length in the water jet cutter coordinate system, position parameters in the linear motion axis of the water jet cutter are an action point jet long-axis position and/or an action point jet long-axis velocity in the water jet cutter coordinates, and position parameters in the rotary motion axis of the water jet cutter are an action point cross-sectional angle in the water jet cutter coordinates.

If position parameters in the jet flow axis of the water jet cutter are acquired in lag time, the motion control module is configured to: subtract and filter a cross-sectional image of the same jet long-axis position obtained in the planning stage and a cross-sectional navigation image at the current moment to obtain a high-frequency image signal; and after performing enhancement and boundary excision of the high-frequency graphic signal, perform measurement to obtain position parameters in the jet flow axis of the water jet cutter.

The embodiments of the present invention provide a high-precision medical automatic water jet cutter with a water jet cutter head that may be configured to eject a high-pressure jet. A pipeline and hydraulic power module includes a high-pressure pipeline that transfers high-pressure brine from the water jet cutter and a suction pipeline that discharges waste liquid to maintain pressure balance within the body. The motion control module includes a cutter head motion control unit, a high-pressure jet pressure control unit, a waste liquid suction control unit and a control host, where the cutter head motion control unit is configured to send position parameters in the linear motion axis and the rotary motion axis to position control elements of the water jet cutter head; the high pressure jet pressure control unit is configured to send position parameters in the jet flow axis to jet flow control elements of the water jet cutter head; the waste liquid suction control unit is configured to send position parameters in the suction flow axis to the pipeline and hydraulic power module; and the control host is configured to receive and convert the motion position trajectory parameters for each axis into motion position control parameters, and communicate with the cutter head motion control unit, the high-pressure jet pressure control unit and the waste liquid suction control unit. An imaging module includes an ultrasound probe and an electric stepper for inserting into a lumen to be examined and obtaining an ultrasound image. An image planning module includes an image planning host that performs planning of the water jet cutter action process based on the navigation image and sends planning parameters to the control host.

According to the high-precision automatic water jet cutter which the present invention relates to, a biplanar ultrasound probe or a three-dimensional ultrasound probe is carried by an electric stepper with precise position feedback, and continuous cross-sectional images obtained when the probe moves with the stepper are saved by taking positions in the motion direction as indexes. When motion trajectory is planned, line-array images of the sagittal plane and the cross-sectional plane implement precise correspondence, thereby avoiding planning errors caused by manually obtaining limited ultrasound images.

The water jet cutter system according to the embodiments of the present invention uses high-precision trajectory planning, for example, the biplanar ultrasound probe is driven by the electric stepper to automatically move for scanning a target and obtaining continuous images of each cross-section of the target in precise correspondence with each position in the sagittal plane, or automatically generating a target envelope based on a three-dimensional image to produce excision parameters. The precision requirements for motion execution are increased accordingly, so the present invention provides a method for unified motion control of the high-pressure jet flow, the suction flow, and mechanical motions in each direction, which coordinates to eliminate errors caused by the influence of various motion axes to each other, and forms closed-loop control based on the actual measurements as motion feedback to improve the control precision. In this way, high-precision operation of the automatic water jet cutter can be implemented, and a fine excision is performed along an actual boundary of the target, thereby achieving a more ideal result.

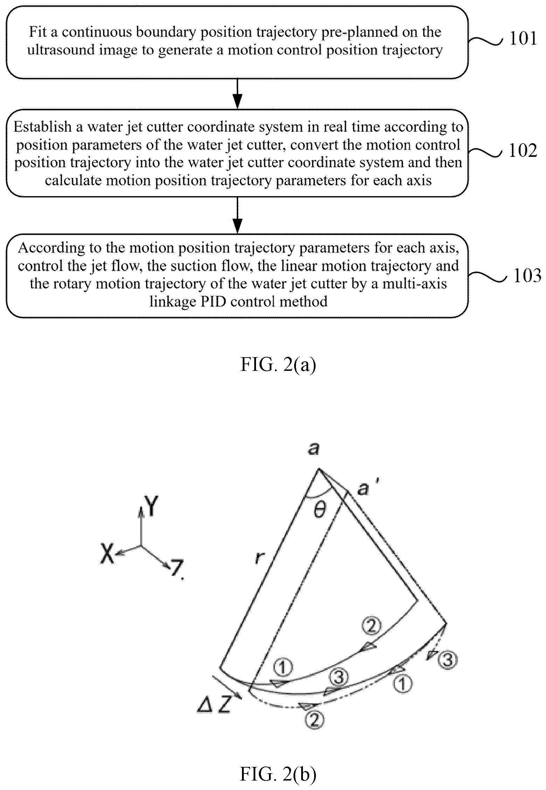

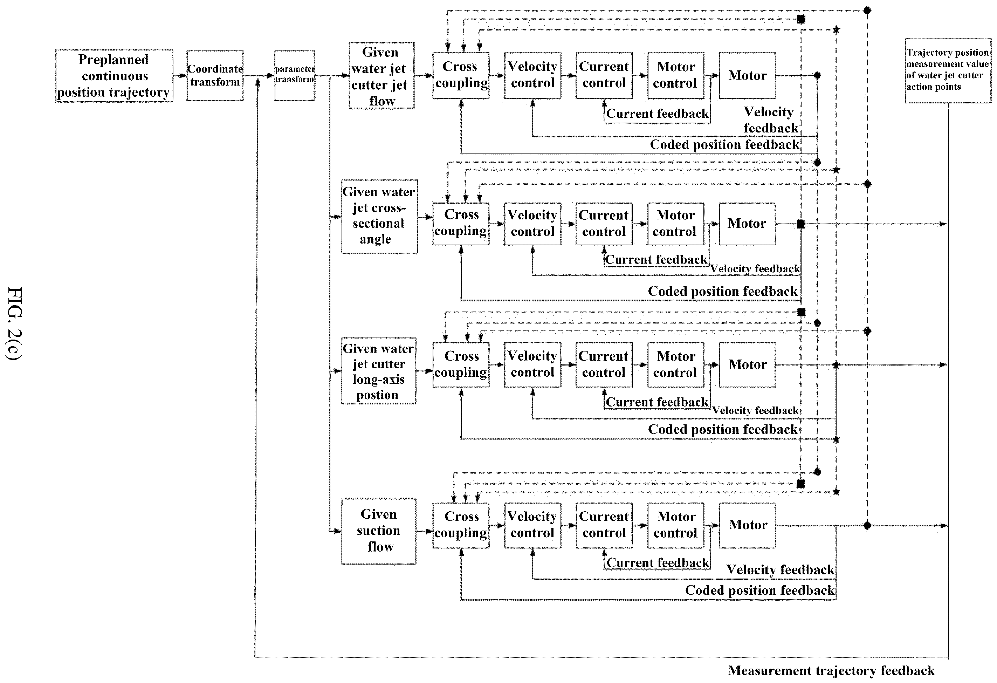

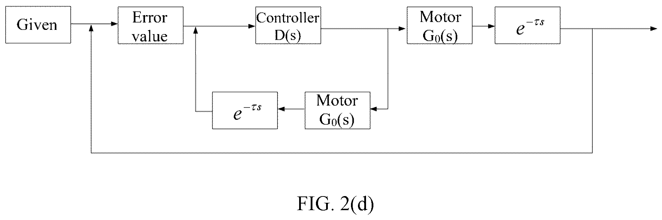

FIG. 2(a) is a method flowchart of a method embodiment of the present application; FIG. 2(b) is a schematic diagram of a Z-shaped excision trajectory of a method embodiment of the present application; FIG. 2(c) is a schematic diagram of PID linkage control of a method embodiment of the present application; and FIG. 2(d) is a schematic diagram of PID time-lag control of a method embodiment of the present application.

The embodiments of the invention can be used to implement linkage control of four axes, namely, the jet flow axis, the suction flow axis, the linear motion axis and the rotary motion axis of the water jet cutter. A water jet cutter automatic control method specifically includes the following steps 101-103.

Step 101: a continuous boundary position trajectory pre-planned on the navigation image is fitted to generate a motion control position trajectory.

It should be noted that the navigation image may be obtained by an ultrasound probe, the navigation image includes a two-dimensional ultrasound image or a three-dimensional ultrasound image, the navigation image may also be obtained by a nuclear magnetic device, the navigation image further includes a three-dimensional image, and the present invention does not limit the specific form of the navigation image.

In the step 101, a continuous boundary position trajectory is fitted to generate a motion control position trajectory, and fitting may be performed with pre-planned key point image information as accurate information, specifically including the following steps 101A to 101C.

Step 101A: a sagittal plane trajectory for the continuous boundary position trajectory is calculated.

In the step 101A, the sagittal plane position may be calculated by a trajectory fitting method of fast multi-axis synchronous minimum error interpolation, and the method serves to reduce as much as possible errors from doctor planned trajectory curves and smooth and soften the motor motion process, while improving the computational efficiency.

In the step 101A, the sagittal plane trajectory obtained by piecewise fitting may be expressed as:

$$\{([y0\_start, z0\_start], [y0\_end, z0\_end]),$$
$$([y1\_start, z1\_start], [y1\_end, z1\_end]),...,$$
$$([yp\_start, zp\_start], [yp\_end, zp\_end])\} \quad (1)$$

where y0_start to yp_start denote y-axis start coordinates of the motion control position trajectories of the $0^{th}$ to $p^{th}$ segments, respectively, obtained by piecewise fitting, and y0_end to yp_end denote y-axis end coordinates of the motion control position trajectories of the $0^{th}$ to $p^{th}$ segments, respectively, obtained by piecewise fitting. z0_start to zp_start denote z-axis start coordinates of the motion control position trajectories of the $0^{th}$ to $p^{th}$ segments, respectively, obtained by piecewise fitting, and z0_end to zp_end denote z-axis end coordinates of the motion control position trajectories of the $0^{th}$ to $p^{th}$ segments, respectively, obtained by piecewise fitting. p+1 is the total number of segments for piecewise fitting, and p is the serial number of the end segment for piecewise fitting.

Step 101B: calculation is performed according to the sagittal plane trajectory and the pre-planned key point image interpolation to obtain the motion control position trajectory.

In the step 101B, considering that planned trajectories for each cross-section are accurate information confirmed by the doctor, and two planned trajectories between the cross-sections are produced by a fitting algorithm such as interpolation, so the key point image is taken as the standard.

When any of the cross-sections occurs within one step size of the sagittal plane trajectory generated in the step 101A, the step size is disassembled, surrounding affected interpolated line segments are recalculated, and the finally generated trajectory is:

$$S_{img} = \{[(y0\_start, z0\_start), (y0\_end, z0\_end), (\theta0\_start, \theta0\_end)],$$
$$......, [(yq\_start, zq\_start), (yq\_end, zq\_end), (\theta q\_start, \theta q\_end)]\} \quad (2)$$

in Equation 2, [(y0_ *start,* z0_ *start),* ( y0_end,z0_end),($\theta$0_end, $\theta$0_end)] denotes one motion voxel, Simg is the motion control position trajectory, $\theta$0_start to $\theta q$_start are start values of cross-sectional angles of motion control position trajectories of the 0th to qth segments obtained by piecewise fitting, and $\theta$0_end to $\theta q$_end are end values of cross-sectional angles of motion control position trajectories of the 0th to qth segments obtained by piecewise fitting. q is the serial number of the end segment after interpolation.

It should be noted that one of the ways of writing Simg is given in Equation 2, and Simg may also be defined according to other orders, which are not specifically limited here.

Step 101C: the motion voxel is interpolated in accordance with a motion trajectory when the water jet cutter is actually operated.

After S_img is obtained, considering that the water jet cutter is required to be a Z-shaped dense excision trajectory to initiate a good excision effect, such as ① to ③ in FIG. 2(b), so it is necessary to perform interpolation and computation of real motion trajectories within each motion voxel.

Specifically, firstly, a distance between two adjacent sides of the Z-shaped trajectory, defined as a pitch c of the Z-shaped trajectory, is obtained according to an experimental excision effect.

Secondly, each motion voxel within the S_img is subdivided by the pitch c, and if a subdivision length of each motion voxel is set to L0, it is required to be subdivided into L0/c segments of the trajectory.

It should be noted that each motion voxel may be subdivided by using the same pitch or different pitches.

Thirdly, a trajectory generated by fitting the subdivided trajectories is used as the motion control position trajectory.

It should be noted that step 101C is an optional step.

Step 102: a water jet cutter coordinate system is established in real time according to position parameters of the water jet cutter, the motion control position trajectory is converted into the water jet cutter coordinate system and then motion position trajectory parameters for each axis are calculated.

In the step 102, the motion position trajectory parameters for each axis include an action point jet length in the water jet cutter coordinate system, an action point cross-sectional angle, and further include an action point jet long-axis position and/or an action point jet long-axis velocity. The action point jet long-axis velocity is a time differential of the action point jet long-axis position.

In the step 102, various parameters obtained from the trajectory planning are sent to the motion control module according to a time interval $\Delta t$, including: an action point cross-sectional angle in the water jet cutter coordinate system in the XY plane, where with an angle of 0 vertically downward along the Y-axis, two ranges on two sides of the action point cross-sectional angle constitute a first angle $\theta_a$ and a second angle $\theta_b$, respectively, in the water jet cutter coordinate system; a rotation direction may be self-defined, for example, a clockwise or counterclockwise direction as observed in a direction from the instrument end towards the target; and a linear motion feed velocity in the Z-direction at any moment, that is, an action point water jet cutter long-axis velocity; and an action point water jet cutter jet length at any moment, and the above parameters determine a desired cavity geometry obtained according to the target boundary. The system makes water jet cutter action end faces of the cavity fit the organ boundary as closely as possible by three-dimensional high-precision planning.

The time interval $\Delta t$ is time required for an origin of the tool coordinate system to rotate $\theta_1$ in one direction in the XY plane, and $\theta_1$ is any of action point cross-sectional angles in the water jet cutter coordinate system. $\Delta t$ may be a fixed value. At this time, the time required to complete a rotation in one direction is equal for different angle sizes, and the average angular velocity corresponding to each point on the rotation trajectory is directly proportional to the angle size. $\Delta t$ may further be a variable. When tissue characteristics require the jet to act on motion voxel points on the trajectory for a specific length of time, the average linear velocity of various points on the trajectory should meet specific requirements, at this time $\Delta t = \theta_1 \times r_1 / v_r$, where $r_1$ also represents a distance between an excision action point of the jet and a nozzle of the water jet cutter, that is, an excision jet length, and $v_r$ is an average linear velocity on the trajectory.

In the step 102, to avoid excessive historical data and calculation, the navigation image trajectory may also be converted by a coordinate transform into a spatial position trajectory in the water jet cutter coordinate system by an incremental control method, that is, a displacement of the origin of the tool coordinate system within each time interval $\Delta t$ is a displacement relative to the position when the coordinate origin is located at an angle of 0 vertically downward within a previous $\Delta t$, then the step of calculating motion position trajectory parameters for each axis further includes:

Step 102A: coordinates of the motion control position trajectory are transformed into the water jet cutter coordinate system to obtain an initial value of the spatial position trajectory in the water jet cutter coordinate system:

$$S_0 = \left[ x_0, y_0, z_0, 1 \right] = \left[ y_{img} \sin \theta_{img}, y_{img}, z_{img}, 1 \right] \quad (3)$$

where $S_0$ is an initial value of the spatial position trajectory in the water jet cutter coordinate system, $x_0$, $y_0$ and $z_0$ are x-axis, y-axis, and z-axis coordinates corresponding to $S_0$, respectively, $y_{img}$, $z_{img}$, and $\theta_{img}$ are y-axis, z-axis, and $\theta$-axis coordinates corresponding to $S_{img}$, respectively, and $y_{img}$ corresponds to $y0\_start$ to $yq\_start$ and $y0\_end$ to $yq\_end$ in Equation 2, and so on.

Step 102B: an excision water jet cutter jet length, an excision water jet cutter cross-sectional angle, and an excision water jet cutter jet long-axis velocity at each moment are acquired in real time to generate first to third transformation matrices:

$$T_x = \begin{bmatrix} 1 & 0 & 0 & \Delta r \sin \Delta \theta \\ 0 & 1 & 0 & 0 \\ 0 & 0 & 1 & 0 \\ 0 & 0 & 0 & 1 \end{bmatrix} \quad (4)$$

$$T_y = \begin{bmatrix} 1 & 0 & 0 & 0 \\ 0 & 1 & 0 & \Delta r \\ 0 & 0 & 1 & 0 \\ 0 & 0 & 0 & 1 \end{bmatrix} \quad (5)$$

$$T_z = \begin{bmatrix} 1 & 0 & 0 & 0 \\ 0 & 1 & 0 & 0 \\ 0 & 0 & 1 & v_{z1}\Delta t \\ 0 & 0 & 0 & 1 \end{bmatrix}_{(6)}$$

where Tx, Ty and Tz are the first, second and third transformation matrices at the current moment, respectively, $\Delta r$ is an increment of the action point jet length at the current moment, which is a difference of the action point jet length between the current moment of acquisition and the previous moment of acquisition, $\Delta\theta$ is an increment of the action point cross-sectional angle at the current moment, which is a difference of the action point cross-sectional angle between the current moment of acquisition and the previous moment of acquisition, $v_{z1}$ is an action point jet long-axis velocity at the current moment, and $\Delta t$ is the time interval, which is a difference of time between the current moment of acquisition and the previous moment of acquisition.

In step 102B, the action point jet long-axis position should be less than or equal to a width of the water jet cutter jet to prevent from excising a portion of the target of which the motion pitch is too large to be covered by the jet. $v_{z1} = z_1 \times v_{r1}/(\theta_1 \times r_1)$ is obtained by $t_1 = z_1/v_{z1}$ and $t_1 = \theta_1 \times r_1/v_{r1}$. Considering that when water jet cutter excision is performed, the jet is required to act on voxel points on the trajectory for a specific length of time to achieve an ideal excision effect, $v_{r1}$ is a reference standard of an excision velocity obtained through experiments. Considering that changes in excision effect may not be a linear relationship at different $\theta_1$, so $v_{r1}$ may also be in the form of a look-up table of a sub-region, and different coefficients are experimentally calibrated according to the excision effect at different $\theta_1$.

Step 102C: an updated value of the spatial position trajectory in the water jet cutter coordinate system is calculated:

$$S_1 = T_x \times T_y \times T_z \times S_0 \quad (7)$$

$$S_1 = [x_1, y_1, z_1, 1] \quad (8)$$

where S1 is an updated value of the spatial position trajectory in the water jet cutter coordinate system at the current moment of acquisition, and x1, y1, and z1 are x-axis, y-axis, and z-axis coordinates corresponding to S1, respectively.

It should be noted that Equation 7 represents a relationship that exists between an updated value of the spatial position trajectory when the spatial position trajectory is updated for the first time and an initial value of the spatial position trajectory. When the updated value of the spatial position trajectory is continuously iterated, the updated value of the spatial position trajectory at the current moment is equal to $T_x \times T_y \times T_z$ multiplied by the updated value of the spatial position trajectory at the previous moment.

Step 102D: motion position trajectory parameters for each axis are calculated according to the spatial position trajectory in the water jet cutter coordinate system:

$$r_1 = y_1 \quad (9)$$

$$\theta_1 = \arcsin(x_1/y_1) \quad (10)$$

In the step 102, the motor motion position parameters may be any one of the following: firstly, the motion position trajectory parameters for each axis include an action point jet length $r_1$, an action point cross-sectional angle $\theta_1$ and an excision jet long-axis position z1 in the water jet cutter coordinate system; secondly, the motion position parameters for each axis include an action point jet length $r_1$, an action point cross-sectional angle $\theta_1$, and an action point jet long-axis velocity vz1 in the water jet cutter coordinate system; and thirdly, the motion position parameters for each axis include an action point jet length $r_1$, an action point cross-sectional angle $\theta_1$, an action point jet long-axis position z1 and an action point jet long-axis velocity vz1 in the water jet cutter coordinate system.

Step 103: according to the motion position trajectory parameters for each axis, the jet flow, the suction flow, the linear motion trajectory and the rotary motion trajectory of the water jet cutter are controlled by a multi-axis linkage PID control method, where the suction flow is equal to the jet flow.

In the step 103, while the water jet cutter jet is operating, the suction pump performs a suction motion by using a strategy of following the jet high pressure pump, and the suction flow is kept dynamically equal to the jet flow.

In the step 103, to ensure that the motion position trajectory parameters for each axis produced in the step 102 may be quickly and smoothly implemented in the motor, linkage PID control of the 4 motion axes is adopted, and control methods such as neural network prediction and other control methods may be added as needed for further optimization, so that position errors for each axis and synchronous position errors for all axes approach to zero as soon as possible, and at the same time, considering the influence of each axis and all the other axes to each other, cross-coupling control of position errors for each axis is performed.

Specifically, position loop feedbacks in the jet flow axis, the suction flow axis, the linear motion axis, and the rotary motion axis are crossed as shown in FIG. 2(c). For the jet flow axis of the water jet cutter, in a jet action distance position loop of the water jet cutter, the control of the jet flow axis is achieved by using PID closed-loop control feedback, and in the independent PID control of the jet flow axis, a current feedback, a velocity feedback and a coded position feedback are performed by the motor. Accordingly, for the rotary motion axis of the water jet cutter, in a cross-sectional angle position loop of the water jet cutter, the control of the rotary motion axis is achieved by using PID closed-loop control feedback, and in an independent PID control of the rotary motion axis, a current feedback, a velocity feedback, and a coded position feedback are performed by the motor. Accordingly, for the linear motion axis of the water jet cutter, in a jet length position loop of the water jet cutter, the control of the linear motion axis is achieved by using PID closed-loop control feedback, and in the independent PID control of the linear motion axis, a current feedback, a velocity feedback, and a coded position feedback are performed by the motor. In the suction flow axis of the water jet cutter, the suction flow is dynamically equal to the jet flow.

Cross-coupling control is also performed in the jet flow axis, the suction flow axis, the linear motion axis and the rotary motion axis, that is, the error of the jet flow axis is synchronously fed back to the jet flow axis, the suction flow axis, the linear motion axis and the rotary motion axis; the error of the suction flow axis is synchronously fed back to the jet flow axis, the suction flow axis, the linear motion axis and the rotary motion axis; the error of the linear motion axis is synchronously fed back to the jet flow axis, the suction flow axis, the linear motion axis and the rotary motion axis; and the error of the rotary motion axis is synchronously fed back to the jet flow axis, the suction flow axis, the linear motion axis and the rotary motion axis.

As shown in FIG. 2(c), the parameter transform refers to converting motion position trajectory parameters for each axis into motion position control parameters, the water jet cutter jet flow refers to the action point jet length in the water jet cutter coordinate system, the water jet cutter cross-sectional angle refers to the action point cross-sectional angle in the water jet cutter coordinate system, the water jet cutter long-axis position refers to the action point jet long-axis position and/or the action point jet long-axis velocity in the water jet cutter coordinate system, and the suction flow refers to the action point jet length in the water jet cutter coordinate system.

A model for cross-coupling control in the jet flow axis, the suction flow axis, the linear motion axis and the rotary motion axis is exemplified below:

$$E_{\theta_1}^{'} = 3E_{\theta_1} - \left(E_{r_1} + E_{z_1} + E_{l_1}\right) \quad (11)$$

$$E_{z_1}^{'} = 3E_{z_1} - \left(E_{r_1} + E_{\theta_1} + E_{l_1}\right) \quad (12)$$

$$E_{r_1}^{'} = 3E_{r_1} - \left(E_{z_1} + E_{\theta_1} + E_{l_1}\right) \quad (13)$$

$$E_{l_1}^{'} = 3E_{l_1} - \left(E_{z_1} + E_{\theta_1} + E_{r_1}\right) \quad (14)$$

where $E_{\theta_1}^{'}$, $E_{z_1}^{'}$, $E_{r_1}^{'}$ and $E_{l_1}^{'}$ are updated values of errors in the rotary motion axis, the linear motion axis, the jet flow axis, and the suction flow axis of the water jet cutter, respectively, and $E_{\theta_1}$, $E_{z_1}$, $E_{r_1}$ and $E_{l_1}$ are error values at the previous moment in the rotary motion axis, the linear motion axis, the jet flow axis, and the suction flow axis of the water jet cutter, respectively.

It should be noted that the above error values for each axis refer to error values of the motion position trajectory parameters for each axis.

In the step 103, in the multi-dimensional linkage PID control method, closed-loop control of a trajectory planning position loop is added, and an error of the trajectory planning loop is a difference between a position on the pre-planned continuous position trajectory at the current moment and an action point trajectory position obtained by measurement at the current moment.

It should be noted that the action point trajectory position refers to motion position trajectory parameters in the linear motion axis, the rotary motion axis, the jet flow axis and the suction flow axis.

A trajectory planning position loop is added besides motion control position loops for each axis. An error of the trajectory planning position loop is a difference between the planned trajectory position and the action point trajectory position obtained by measurement.

In the step 103, the action point trajectory position obtained by measurement at the current moment may be calculated in real time or in lag time.

Preferably, when real-time calculation is used, the action point jet long-axis position and/or the action point jet long-axis velocity and the action point cross-sectional angle and the action point jet length are acquired in real time in the water jet cutter coordinate system to obtain the action point trajectory position.

Specifically, a miniaturized laser range finder in synchronized motion with a nozzle is installed in the water jet cutter head, such as a miniaturized laser range finder module with a millimeter-scale outer diameter using a TOF (time of flight or femtosecond) technology, to measure a distance from the water jet cutter to a cavity boundary of the excised target object while the water jet cutter jet is operating and to obtain the action point trajectory coordinates by the first to the third transformation matrices of the coordinate system conversion matrices. In the coordinate conversion, $r_1$ is a distance actually determined by the range finder, and $\theta_1$, $z_1$, $v_{z1}$ and the like are obtained from feedbacks of each motor. The distance from the water jet cutter to the cavity boundary of the excised target object may also be obtained through a miniaturized ultrasonic hydrophone, a binocular three-dimensional stereoscopic endoscopic image and other technical methods.

Preferably, when lagged calculation is used, the water jet cutter action point jet action distance in the water jet cutter coordinate system acquired in lag time by a cross-sectional image is combined with the water jet cutter an action point cross-sectional angle and the water jet cutter action point jet length acquired in real time, to perform closed-loop control of a trajectory planning position loop by a time-lag system PID regulation method.

Specifically, based on a biplanar ultrasound probe carried by an electric stepper, while a water jet cutter excision operation is performed, the double-sided ultrasound probe is carried by the electric stepper and moves along the long-axis z direction of the probe in synchronization with a linear motion of the water jet cutter in the Z direction, while the probe performs the continuous acquisition of cross-sectional images. Since the probe cross-sectional line array is located at a fixed distance from the nozzle of the water jet cutter in the -Z direction, an excision depth $r_1$ measured from the cross-sectional image is a linearly delayed measurement feedback lagging behind the real-time trajectory planning input Δt, that is, the feedback sampling is asynchronous with the control volume sampling. At this point, the existing control process may be optimized by a time-lag system PID regulation method, for example, using Smith prediction control, fuzzy control and the like.

As shown in FIG. 2(d), as an example of using the Smith predictive control method, the motor characteristic transfer equation is Laplace-transformed to be expressed as G0(s)e-τs, where the delay time is transformed to e-τs, and this transfer equation is added in reverse parallel to the controller to form a new Smith prediction control flow.

In the step 103, if the time-lag PID control is used, measurement of the excision depth may be performed automatically by using an algorithm for the cross-sectional image: the pre-planned cross-sectional navigation image of the same position and the cross-sectional navigation image at the current moment are subtracted and filtered to obtain a high-frequency image signal; and enhancement of the high-frequency graphic signal is performed to obtain an excision boundary, and then the water jet cutter jet action distance in the water jet cutter coordinate system is obtained by measurement.

Specifically, since the ultrasound probe is carried by the electric stepper to move, the cross-sectional image acquired at this point and the cross-sectional image acquired prior to the aforementioned trajectory planning are both precisely positioned images with position indexes. The excision depth automatic measurement algorithm may use images of the same position before planning and after water jet cutter excision to be subtracted as image preprocessing, and a high-frequency image signal obtained after filtering of the subtraction result embodies changes in the cross-sectional image caused by the excision action of the water jet cutter. An excision boundary obtained by the enhancement of the image at this point is measured to obtain a measured value.

To implement parameter settings for high-precision trajectory planning, the water jet cutter system in the embodiment of the present invention employs unified linkage control of multiple motions to improve the control precision. This water jet cutter system is configured to excise irregularly shaped target objects, requiring the cutter head to reciprocate and rotate at a given angle in a plane perpendicular to the cutter body while moving in a straight line in the direction of the cutter body, and adjusting the high-pressure jet flow and pressure according to the changes in boundary shapes of the target objects to control the excision action distance. At the same time, according to the changes in pressure caused by the changes in high-pressure jet flow, the suction flow of the suction unit is adjusted, and the waste liquid is discharged to maintain the pressure balance. From the perspective of motion control, above linear motion in the direction of the cutter body, rotary motion in the plane perpendicular to the cutter body, high-pressure jet flow pressure adjustment motion and suction flow change adjustment motion may be regarded as four axial motions. Non-synchronization of the 4 axial motions leads to problems such as reduced excision accuracy and pressure imbalance. To ensure the synchronization of various axial motions, the embodiments of the present invention use the motion trajectory of the coordinate system origin of the jet action tool to uniformly represent motions for each axis, and the control program carries out linkage control of position loops for each axis through this motion trajectory, and at the same time, a method of measuring the actual motion trajectory as a feedback to form closed-loop control is provided, thereby improving the operating precision of the water jet cutter.

Those skilled in the art should understand that embodiments of the present invention may be provided as a method, a system, or a computer program product. Therefore, the present invention may use a form of hardware only embodiments, software only embodiments, or embodiments with a combination of software and hardware. In a typical configuration, a device of the present application includes one or more processors (one of CPU, FGAP and MUC), input/output user interfaces, network interfaces and memories.

Therefore, the present application further provides an electronic device, including: a memory, a processor, and a computer program stored in the memory and capable of running in the processor, where the computer program, when executed by the processor, implements the method described in any one of the embodiments of the first aspect of the present application.

Moreover, the present application may use a form of a computer program product that is implemented on one or more computer-usable storage media (including but not limited to a disk memory, a CD-ROM, an optical memory and the like) that include computer-usable program code.

Therefore, the present application further provides a computer-readable medium, where the computer-readable medium stores a computer program; and when the computer program is executed by a processor, the steps of the method according to any embodiment of the present application are implemented. For example, the memory of the present invention may include a non-permanent memory, a random access memory (RAM), a non-volatile memory and/or the like in the computer-readable medium, such as a read-only memory (ROM) or a flash RAM.

The computer-readable medium includes permanent and non-permanent, removable and non-removable media, and may store information by using any method or technology. The information may be computer-readable instructions, data structures, modules of programs or other data. Examples of the computer storage medium include but not limited to: a phase-change memory (PRAM), a static random access memory (SRAM), a dynamic random access memory (DRAM), another type of random access memory (RAM), a read-only memory (ROM), an electrically erasable programmable read-only memory (EEPROM), a flash memory or another memory technology, a compact disc read-only memory (CD-ROM), a digital video disc (DVD) or another optical memory, a magnetic cassette, a magnetic disk storage, another magnetic storage device, and any other non-transmission medium that may be configured to store information that can be accessed by a computing device. As defined in this specification, the computer-readable medium does not include computer-readable transitory media, such as modulated data signals and carriers.

It should be noted that the terms "comprise", "include" and any other variants thereof are intended to cover non-exclusive inclusion, so that a process, a method, a commodity or a device that includes a series of elements not only includes these very elements, but may further include other elements not expressly listed, or further include elements inherent to this process, method, commodity or device. In the absence of more limitations, an element defined by "include a..." does not exclude other same elements existing in the process, method or device including the element.

The above are only embodiments of the present invention and are not intended to limit the present invention. For a person skilled in the art, various changes and variations may be made in the present invention. Any modifications, equivalent substitutions, improvements and the like made within the spirit and principle of the present invention should be included within the scope of the claims of the present invention.

## Claims

1.    An automatic water jet cutter system, comprising:

    an imaging module, configured to generate a navigation image;
    an image planning module, configured to fit a continuous boundary position trajectory pre-planned on the navigation image to generate a motion control position trajectory, and convert the motion control position trajectory into a water jet cutter coordinate system, then perform calculation and send motion position trajectory parameters for each axis corresponding to water jet cutter jet action points;
    a motion control module, configured to receive the motion position trajectory parameters for each axis, generate and send motion position trajectory parameters in a linear motion axis and a rotary motion axis to a water jet cutter head by a multi-axis linkage control method, and send motion position control parameters in a jet flow axis and a suction flow axis to a pipeline and hydraulic power module, and further configured to acquire the motion position trajectory parameters for each axis of the water jet cutter head for closed-loop control of the motion trajectory;

a pipeline and hydraulic power module, configured to transfer liquid to the water jet cutter head according to the motion position control parameters in the jet flow axis sent by the motion control module and perform a suction motion according to the motion position control parameters in the suction flow axis; and

a water jet cutter head, configured to perform respective motions according to the motion position control parameters in the linear motion axis and the rotary motion axis sent by the motion control module, and the motion position control parameters in the jet flow axis,

wherein in the multi-axis linkage control method, an error in any one of the jet flow axis, the suction flow axis, the linear motion axis and the rotary motion axis of the water jet cutter are correlated with errors in the other three axes.

2. The automatic water jet cutter system according to claim 1, wherein the motion control position trajectory comprises a jet effective length, a jet long-axis position and a cross-sectional angle; and the motion position trajectory parameters for each axis comprise an action point jet length in the water jet cutter coordinate system, an action point cross-sectional angle, and further comprises an action point jet long-axis position and/or an action point jet long-axis velocity.

3. The automatic water jet cutter system according to claim 1, wherein in the multi-axis linkage control method, closed-loop control of a trajectory planning position loop is added, and an error value of the trajectory planning position loop is a difference between a boundary position on the continuous boundary position trajectory at the current moment of measurement and an action point trajectory position obtained by actual measurement.

4. The automatic water jet cutter system according to claim 1, wherein in the multi-axis linkage control method, errors in the jet flow axis, the suction flow axis, the linear motion axis and the rotary motion axis of the water jet cutter are expressed as:

$$E'_{\theta_1} = 3E_{\theta_1} - \left( E_{r_1} + E_{z_1} + E_{l_1} \right)$$

$$E'_{z_1} = 3E_{z_1} - \left( E_{r_1} + E_{\theta_1} + E_{l_1} \right)$$

$$E'_{r_1} = 3E_{r_1} - \left( E_{z_1} + E_{\theta_1} + E_{l_1} \right)$$

$$E'_{l_1} = 3E_{l_1} - \left( E_{z_1} + E_{\theta_1} + E_{r_1} \right)$$

wherein $E'_{\theta_1}$, $E'_{z_1}$, $E'_{r_1}$ and $E'_{l_1}$ are updated values of errors in the rotary motion axis, the linear motion axis, the jet flow axis, and the suction flow axis of the water jet cutter, respectively, and $E'_{\theta_1}$, $E'_{z_1}$, $E'_{r_1}$ and $E'_{l_1}$ are error values at the previous moment in the rotary motion axis, the linear motion axis, the jet flow axis, and the suction flow axis of the water jet cutter, respectively.

5. The automatic water jet cutter system according to claim 1, wherein a miniaturized laser range finder or hydrophone in synchronized motion with a nozzle is installed in the water jet cutter head for obtaining the action point jet long-axis position of the water jet cutter in real time.

6. The automatic water jet cutter system according to claim 1, wherein the imaging module further comprises: an electric stepper and an imaging probe; the electric stepper is configured to drive the imaging probe to move to obtain navigation images of various positions.

7. The automatic water jet cutter system according to claim 1, wherein the imaging module further comprises: a biplanar ultrasound probe or a three-dimensional ultrasound probe;
the biplanar ultrasound probe or the three-dimensional ultrasound probe is configured to obtain ultrasound images; and the biplanar ultrasound probe is configured to obtain biplanar ultrasound images, and the three-dimensional ultrasound probe is configured to obtain three-dimensional ultrasound images.

8. The automatic water jet cutter system according to claim 1, wherein the imaging module further comprises: a nuclear magnetic resonance image module, configured to obtain a three-dimensional image.

9. The automatic water jet cutter system according to claim 1, wherein the image planning module is configured to perform piecewise fitting of the continuous boundary position trajectory to generate motion control position trajectories for each segment combined to form the motion control position trajectory.

10. The automatic water jet cutter system according to claim 1, wherein the image planning module is further configured to convert the motion control position trajectory into a spatial position trajectory in the water jet cutter coordinate system through a coordinate transform and by an incremental control method, and then calculate motion position trajectory parameters for each axis.

11. The automatic water jet cutter system according to claim 1, wherein the image planning module is configured to take key point image positions on a pre-designed navigation image as accurate information during fitting.

12. The automatic water jet cutter system according to claim 1, wherein the image planning module is further configured to interpolate each of the motion voxels in accordance with a motion trajectory when the water jet cutter is actually operated so as to generate the motion control position trajectory.

13. The automatic water jet cutter system according to claim 3, wherein the motion control module is configured to acquire motion position parameters in the jet flow axis, the linear motion axis and the rotary motion axis of the water jet cutter and positions of the continuous boundary position trajectory in the water jet cutter coordinate system in real time, and subtract corresponding coordinates to obtain an error value of the trajectory planning position loop.

14. The automatic water jet cutter system according to claim 3, wherein the motion control module is configured to combine the motion position trajectory parameters in the jet flow axis of the water jet cutter acquired in lag time by a cross-sectional image with the motion position trajectory parameters in the rotary motion axis and the linear motion axis of the water jet cutter acquired in real time, to perform closed-loop control of a trajectory planning position loop by a time-lag system regulation method.

15. The automatic water jet cutter system according to claim 9, wherein the image planning module is further configured to perform piecewise fitting of the continuous boundary position trajectory by using stepped line segments to obtain the motion control position trajectories for each segment.

16. The automatic water jet cutter system according to claim 9, wherein the image planning module is further configured to perform piecewise fitting of the continuous boundary position trajectory by a straight line segment interpolation fitting method to obtain the motion control position trajectories for each segment.

17. The automatic water jet cutter system according to claim 14, wherein the motion control module is configured to subtract and filter a cross-sectional image of the same position acquired in advance and a cross-sectional navigation image acquired at the current moment, to obtain a high-frequency image signal; and after performing enhancement and boundary excision of the high-frequency graphic signal, perform measurement to obtain the motion position trajectory parameters in the jet flow axis of the water jet cutter.

1          2          4          5

| Imaging module | Image planning module | Water jet cutter head | Pipeline and hydraulic power module |

3

| Motion control module |

FIG. 1(a)

FIG. 1(b)

FIG. 1(c)

FIG. 1(d)

FIG. 1(e)

FIG. 1(f)

FIG. 1(g)

FIG. 1(h)

Fit a continuous boundary position trajectory pre-planned on the ultrasound image to generate a motion control position trajectory — 101

Establish a water jet cutter coordinate system in real time according to position parameters of the water jet cutter, convert the motion control position trajectory into the water jet cutter coordinate system and then calculate motion position trajectory parameters for each axis — 102

According to the motion position trajectory parameters for each axis, control the jet flow, the suction flow, the linear motion trajectory and the rotary motion trajectory of the water jet cutter by a multi-axis linkage PID control method — 103

FIG. 2(a)

FIG. 2(b)

FIG. 2(c)

FIG. 2(c)

FIG. 2(d)

<div align="center">INTERNATIONAL SEARCH REPORT</div>

| International application No. |
|---|
| **PCT/CN2023/090360** |

| A. | CLASSIFICATION OF SUBJECT MATTER |
|---|---|

A61B17/3203(2006.01)i; A61B34/10(2016.01)i; A61B34/20(2016.01)i

According to International Patent Classification (IPC) or to both national classification and IPC

| B. | FIELDS SEARCHED |
|---|---|

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CNTXT; ENTXTC; ENTXT; OETXT; VEN; CNKI; IEEE: 北京智愈, 水刀, 射流, 切, 割, 轴, 联动, 自动, 导航, 规划, 闭环, 直线, 旋转, 轨迹, 精度, PID, knife, cut+, axis, axes, water, jet, track, automatic, linkage, motion, rotat+, rectilinear, closed loop, precision

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 114521939 A (HEALINNO (BEIJING) MEDICAL TECHNOLOGY CO., LTD.) 24 May 2022 (2022-05-24)<br>claims 16-32, and description, paragraphs [0003]-[0140], and figures 1(a)-2(d) | 1-17 |
| PX | CN 115444513 A (HEALINNO (BEIJING) MEDICAL TECHNOLOGY CO., LTD.) 09 December 2022 (2022-12-09)<br>description, paragraphs [0004]-[0163], and figures 1(a)-2(d) | 1-17 |
| A | CN 105764436 A (PROCEPT BIOROBOTICS CORPORATION) 13 July 2016 (2016-07-13)<br>description, paragraphs [0011]-[0520], and figures 1-77 | 1-17 |
| A | CN 114376610 A (HEALINNO (BEIJING) MEDICAL TECHNOLOGY CO., LTD.) 22 April 2022 (2022-04-22)<br>entire document | 1-17 |
| A | CN 103809520 A (SHENYANG INSTITUTE OF COMPUTING TECHNOLOGY CO., LTD., CHINESE ACADEMY OF SCIENCES) 21 May 2014 (2014-05-21)<br>entire document | 1-17 |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **31 July 2023** | **14 August 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2023/090360** |

**C.    DOCUMENTS CONSIDERED TO BE RELEVANT**

| Category* | Citation of document, with indication, where appropriate, of the relevant passages | Relevant to claim No. |
|---|---|---|
| A | CN 109782815 A (XI'AN JIAOTONG UNIVERSITY) 21 May 2019 (2019-05-21)<br>entire document | 1-17 |
| A | US 2019282245 A1 (MAKO SURGICAL CORP.) 19 September 2019 (2019-09-19)<br>entire document | 1-17 |
| A | US 2006156875 A1 (DEPUY MITEK, INC.) 20 July 2006 (2006-07-20)<br>entire document | 1-17 |

Form PCT/ISA/210 (second sheet) (July 2022)

INTERNATIONAL SEARCH REPORT
Information on patent family members

International application No.

**PCT/CN2023/090360**

| Patent document cited in search report | | | Publication date (day/month/year) | Patent family member(s) | | | Publication date (day/month/year) |
|---|---|---|---|---|---|---|---|
| CN | 114521939 | A | 24 May 2022 | None | | | |
| CN | 115444513 | A | 09 December 2022 | None | | | |
| CN | 105764436 | A | 13 July 2016 | None | | | |
| CN | 114376610 | A | 22 April 2022 | None | | | |
| CN | 103809520 | A | 21 May 2014 | None | | | |
| CN | 109782815 | A | 21 May 2019 | None | | | |
| US | 2019282245 | A1 | 19 September 2019 | US | 11457932 | B2 | 04 October 2022 |
| US | 2006156875 | A1 | 20 July 2006 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210433774